# EUROPEAN PATENT APPLICATION

(11) **EP 4 166 531 A1**
(43) Date of publication of application: **19.04.2023**
(21) Application number: 21202163.8
(22) Date of filing: 12.10.2021
(51) Int. Cl.: C07C 37/02, C07C 39/04, C07C 39/28, C07C 39/27, C07C 39/24, C07C 39/06, C07C 39/15, C07C 39/12, C07C 41/26, C07C 43/23, C07C 45/64, C07C 49/825, C07C 49/747, C07C 67/31, C07C 69/86, C07C 253/30, C07C 255/53, C07C 319/20, C07C 315/04, C07C 317/22, C07C 69/94

(54) **PROCESS FOR PREPARING A PHENOLIC HYDROCARBON**

(71) Applicant: Studiengesellschaft Kohle mbH, 45470 Mülheim (DE)
(72) Inventor: CORNELLA, Josep, 45468 Mülheiman der Ruhr (DE); LE VAILLANT, Franck, 78000 Versailles (FR)

(57) **Abstract**

The present invention discloses a distinct mode of action of a nickel catalyst to forge C―O bonds to unlock the mild utilization of N₂O as O-atom transfer reagent in the synthesis of complex phenols from the corresponding aryl halides.

## Description

The present invention is directed to a process for preparing a phenolic hydrocarbon starting from an aryl halogenide and making use of N₂O as oxygen source.

The increasing emission of greenhouse gases represents a global environmental threat, and strategies to address this major issue have been the focus of intense research efforts in recent times. From the sustainability point of view, the development of chemical processes that extend beyond the traditional degradations and repurpose such gaseous byproducts as useful synthons to produce valuable chemical feedstocks is a highly coveted approach.

While the revalorization of CO₂ or CH₄ as C1 sources for organic synthesis through catalytic strategies has received a great deal of attention, much less interest has been placed in the chemical transformation of another major contributor to the global warming: nitrous oxide (N₂O). Governmental reports together with recent scientific evidence coincide in alerting about the consequences that result from the increasing presence of this undervalued gas in the atmosphere. N₂O exhibits a remarkable global warming potential of >300 times that of CO₂, with a decomposition half-life in the atmosphere of >100 years.

Human activities have accelerated the emissions, with an estimated increase rate of N₂O of 2% per decade. Yet, a detailed analysis through the lens of sustainable synthesis presents a unique opportunity for N₂O revalorization, as it represents an excellent O-atom source: it is readily available, non-toxic (laughing gas), and releases benign N₂ as byproduct upon O removal.

Conversely, N₂O is a kinetically inert gas, whose activation require high temperatures (140-350 °C) and pressures (50-200 bar), resulting in limited applications as oxidant for organic synthesis. Yet, the structure of N₂O has captivated chemists, who studied in detail coordination modes and reactivity thereof in a plethora of metal complexes. However, few reports focused on its activation toward the formation of C-O bonds; arguably, among the most valuable bonds in organic synthesis, as it would permit access to highly coveted alcohols, ethers, epoxides, etc. Still, these occasional examples rely on traditional metal-oxo reactivity, which requires high temperatures (100-200 °C) and pressures (10 bar) or long reaction times (1 TON/week).

In a groundbreaking report, Hillhouse (J. Am. Chem. Soc. 109, 5538-5539 (1987)) observed a fundamentally different outcome: upon exposure to N₂O atmosphere, the O atom could be inserted into a Hf-Ph bond of 1 forging the desired Hf-O-Ph (2) with concomitant extrusion of N₂ as illustrated in the Figures. However, regioselectivity issues arose, as the O atom was competitively transferred to the hydride ligand, thus also producing a Hf-O-H complex (3). Mechanistic studies on the O insertion step between various M-C(sp²) bonds using N₂O, peroxides or oxygen suggest that an organometallic Baeyer-Villiger-type (OMBV) mechanism is operating, where the anionic carbon migrates to the encounter of the coordinated O atom, forging the M-O-C bond.

Based on these findings, the inventors aimed at unlocking the potential of N₂O as O-atom source *in a catalytic system,* toward a fundamentally different synthesis of phenols. In contrast to the traditional synthesis, after C-O bond formation the oxidation state of the metal center remains intact, thus requiring an external reductant to close the cycle.

To orchestrate such reductive process, the inventors focused their attention on Ni and its demonstrated ability to maneuver between different oxidation states through SET. In the present invention, the inventors demonstrate that a mechanistically guided approach to activation of N₂O with organometallic complexes results in the development of a mild and selective catalytic synthesis of high value phenols from aryl halides using N₂O as electrophilic O-source. The remarkably mild conditions (25 °C and 1.5 - 2 atm) allow the accommodation of a variety of functional groups, including base-sensitive moieties, thus providing an orthogonal strategy to the current technologies as illustrated in the Figures. This catalytic protocol represents a radically distinct approach for the synthesis of C-O bonds and provides a solution to the mild revalorization of N₂O as a benign O source for synthesis.

Thus, the present invention is directed to a process for preparing a phenolic hydrocarbon wherein an aryl halogenide is reacted with N₂O, alkali iodide selected from sodium or potassium iodide, and Zn in the presence of a Ni-complex having a ligand selected from bipy-pyr and terpy, in a temperature range of 0 °C to 50 °C whereby the halogenide on the aryl moiety is replaced by hydroxyl.

The inventive process can be applied to a broad number of aryl halogenides including branched and polycyclic aryl or heteroaryl compounds, and the aryl or heteroaryl halogenide is usually selected from the aryl or heteroaryl bromide or aryl iodide. The aryl moiety, i.e. the aryl residue of the aryl halogenide, can be any aryl moiety, such as benzyl, naphthyl, anthracenyl, biphenyl, fluorenyl, indanolyl, thianthrenyl, quinolyl, and further aryl or heteroaryl moieties.

In more detail, the aryl halogenide is preferably selected from:
- monocyclic or polycyclic aromatic hydrocarbons having 6 to 22 carbon atoms,
- monocyclic or polycyclic heteroaromatic hydrocarbons having 5 to 21 carbon atoms, or
- monocyclic or polycyclic aralkyl hydrocarbons having 6 to 34 carbon atoms, each of which hydrocarbons may be substituted by one or more substituents selected from -OR, -F, -CI, -Br, -I, -NR₂, -CF₃, -CN, -(C=O)-R, -(C=O)-O-R', -(C=O)-NR₂,-(P=O)(OR'))_{2,}, -SO₂R', -SR, -SF₅, -B(OR')₂, wherein R represents hydrogen, an aliphatic hydrocarbon residue having 1 to 12 carbon atoms and wherein R' represents an aliphatic hydrocarbon residue having 1 to 12 carbon atoms.

The reaction solvent is usually a polar aprotic solvent such as *N,N*-dimethylacetamide, *N,N-*dimethylformamide, THF, etc.

The Ni-complex is prepared in situ in an aprotic polar solvent such as *N,N-*dimethylacetamide from a Ni halogenide such as NiBr₂ or NiI₂ (or the corresponding glyme and diglyme adducts) which are then complexed with a ligand (L) selected from bipy-pyr and terpy. A preformed adduct of the LNiBr₂ or LNiCl₂ is also a competent pre-catalyst.

The inventors have investigated the role of the ligand for the Ni-complex used in the inventive process in detail. Specifically, the inventors turned their attention to the synthesis of electronically asymmetric tridentate ligands. Using only one pyrazolyl allowed the identification of bipyridine-pyrazolyl as an excellent candidate for the catalytic synthesis of phenols using N₂O and aryl halides. Surprisingly, the homocoupling is dramatically suppressed. Other scaffolds, involving protic side arm-, nitrile containing, more flexible or more rigid ligands, resulted in diminished performance, albeit yield of phenol can be observed.

Fine-tuning the bipyridine-pyrazolyl scaffold did not improve the yields further, but still provided useful information on the important parameters to consider on the pyrazolyl moiety: 1) steric hindrance close to the Ni center is detrimental; 2) EWG in the heterocycles is detrimental; 3) EDG is tolerated and disubstitution is also tolerated as long as it is not close to the Ni center; 4) pyrazolyl scaffold is superior to triazene or thiophene. Thus, the ligands bipy-pyr and terpy are preferred.

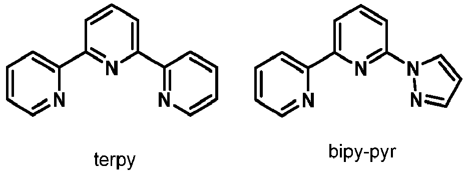

Usually, the inventive process is carried out in a purified and dried reaction vessel under a N₂O-pressure higher than atmospheric pressure, sufficiently in the range of > 1,0 to 2,5 bar, preferably in the range of 1,5 to 2,0 bar, more preferably in the range of 1,9 to 2,0 bar.

The reaction time and reaction temperature are not critical and are in the range 12 to 36 hours, preferably 18 to 30 hours and in the range of 0 °C to 50 °C, preferably from 15 °C to 30 °C, respectively.

### Definitions

For the terms used in the present text, the following definitions apply. The term "aliphatic" includes both saturated and unsaturated, straight chain (*i.e.,* unbranched), branched, acyclic, cyclic, or polycyclic aliphatic hydrocarbons, which are optionally substituted with one or more functional groups. As will be appreciated by one of ordinary skill in the art, "aliphatic" is intended herein to include, but is not limited to, alkyl, alkenyl, alkynyl, cycloalkyl, cycloalkenyl, and cycloalkynyl moieties. Thus, the term "alkyl" includes straight, branched and acyclic alkyl groups. An analogous convention applies to other generic terms such as "alkenyl", "alkynyl", and the like. Furthermore, the terms "alkyl", "alkenyl", "alkynyl", and the like encompass both substituted and unsubstituted groups. In certain embodiments, "lower alkyl" is used to indicate those alkyl groups (acyclic, substituted, unsubstituted, branched or unbranched) having 1-6 carbon atoms.

As used herein, "alkyl" refers to a radical of a straight-chain or branched saturated hydrocarbon group having from 1 to 20 carbon atoms ("C₁₋₂₀ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include methyl (C₁), ethyl (C₂), n-propyl (C₃), isopropyl (C₃), n-butyl (C₄), tert-butyl (C₄), sec-butyl (C₄), iso-butyl (C₄), n-pentyl (C₅), 3-pentanyl (C₅), amyl (C₅), neopentyl (C₅), 3-methyl-2-butanyl (C₅), tertiary amyl (C₅), and n-hexyl (C₆). Additional examples of alkyl groups include n-heptyl (C₇), n-octyl (C₈) and the like. Unless otherwise specified, each instance of an alkyl group is independently unsubstituted (an "unsubstituted alkyl") or substituted (a "substituted alkyl") with one or more substituents. In certain embodiments, the alkyl group is an unsubstituted C₁₋₁₀ alkyl (*e.g*., -CH₃). In certain embodiments, the alkyl group is a substituted C₁₋₁₀ alkyl.

When a range of values is listed, it is intended to encompass each value and sub-range within the range. For example "C₁₋₆" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆.

"Aryl" refers to a radical of a monocyclic or polycyclic (e.g., bicyclic or tricyclic) 4n+2 aromatic ring system (e.g., having 6, 10, or 14 pi electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; e.g., phenyl). In some embodiments, an aryl group has ten ring carbon atoms ("C₁₀ aryl"; e.g., naphthyl such as 1-naphthyl and 2-naphthyl). In some embodiments, an aryl group has fourteen ring carbon atoms ("C₁₄ aryl"; e.g., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently optionally substituted, *i.e.,* unsubstituted (an "unsubstituted aryl") or substituted (a "substituted aryl") with one or more substituents. In certain embodiments, the aryl group is unsubstituted C₆₋₁₄ aryl. In certain embodiments, the aryl group is substituted C₆₋₁₄ aryl.

"Aralkyl" is a subset of alkyl and aryl and refers to an optionally substituted alkyl group substituted by an optionally substituted aryl group. In certain embodiments, the aralkyl is optionally substituted benzyl. In certain embodiments, the aralkyl is benzyl. In certain embodiments, the aralkyl is optionally substituted phenethyl. In certain embodiments, the aralkyl is phenethyl.

"Heteroaryl" refers to a radical of a 5-14 membered monocyclic or bicyclic 4n+2 aromatic ring system (e.g., having 6 or 10 pi electrons shared in a cyclic array) having ring carbon atoms and 1-4 ring heteroatoms provided in the aromatic ring system, wherein each heteroatom is independently selected from nitrogen, oxygen, and sulfur ("5-14 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (e.g., indolyl, quinolinyl, carbazolyl, and the like) the point of attachment can be on either ring, *i.e.,* either the ring bearing a heteroatom (*e.g.,* 2-indolyl) or the ring that does not contain a heteroatom (e.g., 5-indolyl).

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur.

Unless otherwise specified, each instance of a heteroaryl group is independently optionally substituted, *i.e.,* unsubstituted (an "unsubstituted heteroaryl") or substituted (a "substituted heteroaryl") with one or more substituents. In certain embodiments, the heteroaryl group is unsubstituted 5-14 membered heteroaryl. In certain embodiments, the heteroaryl group is substituted 5-14 membered heteroaryl.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

"Heteroaralkyl" is a subset of alkyl and heteroaryl and refers to an optionally substituted alkyl group substituted by an optionally substituted heteroaryl group.

"Unsaturated" or "partially unsaturated" refers to a group that includes at least one double or triple bond. A "partially unsaturated" ring system is further intended to encompass rings having multiple sites of unsaturation, but is not intended to include aromatic groups (e.g., aryl or heteroaryl groups) as herein defined. Likewise, "saturated" refers to a group that does not contain a double or triple bond, *i.e.,* contains all single bonds.

Alkyl, alkenyl, alkynyl, carbocyclyl, heterocyclyl, aryl, and heteroaryl groups, which are divalent bridging groups, are further referred to using the suffix -ene, e.g., alkylene, alkenylene, alkynylene, carbocyclylene, heterocyclylene, arylene, and heteroarylene.

An atom, moiety, or group described herein may be unsubstituted or substituted, as valency permits, unless otherwise provided expressly. The term "optionally substituted" refers to substituted or unsubstituted.

Alkyl, alkenyl, alkynyl, carbocyclyl, heterocyclyl, aryl, and heteroaryl groups are optionally substituted (e.g., "substituted" or "unsubstituted" alkyl, "substituted" or "unsubstituted" alkenyl, "substituted" or "unsubstituted" alkynyl, "substituted" or "unsubstituted" carbocyclyl, "substituted" or "unsubstituted" heterocyclyl, "substituted" or "unsubstituted" aryl or "substituted" or "unsubstituted" heteroaryl group). In general, the term "substituted", whether preceded by the term "optionally" or not, means that at least one hydrogen present on a group (*e.g.,* a carbon or nitrogen atom) is replaced with a permissible substituent, *e.g.,* a substituent which upon substitution results in a stable compound, e.g., a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. For purposes of this invention, heteroatoms such as nitrogen may have hydrogen substituents and/or any suitable substituent as described herein which satisfy the valencies of the heteroatoms and results in the formation of a stable moiety. In certain embodiments, the substituent is a carbon atom substituent. In certain embodiments, the substituent is a nitrogen atom substituent. In certain embodiments, the substituent is an oxygen atom substituent. In certain embodiments, the substituent is a sulfur atom substituent.

A heterosubstituent as defined according to the invention can be selected from OH, F, Cl, Br, I, CN, NO₂, I-R^{S}₂, NO, NCO, -NCS, -SCN, SO₃H, a monohalogenomethyl group, a dihalogenomethyl group, a trihalogenomethyl group, CF(CF3)₂, SF₅, aliphatic, aromatic, heteroaromatic, primary, secondary, tertiary amine or ammonium bound through N atom,-O-alkyl (alkoxy), -O-aryl, -O-heteroaryl -O-SiR^{S}₃,-S-S-R^{S}, -S-R^{S}, -S(O)-R^{S}, -S(O)₂-R^{S},-COOH, -CO₂-R^{S}, -BR^{S}₂, -PR^{S}₂, -OPR^{S}₂, amide, bound through C or N atom, formyl group,-C(O)-R^{S}, -COOM, where M is a metal such as Li, Na, K, Cs, Ag. R^{S} may be, independently from each other, the same or different and is each an aliphatic, heteroaliphatic, aromatic or heteroaromatic group, each optionally being further substituted by one or more heterosubstituents, aliphatic, heteroaliphatic, aromatic or heteroaromatic groups; and/or optionally bridged by an -O- atom, represents a halogenide

The present inventions is further illustrated by the Figures and Examples. In the Figures, it is shown in:

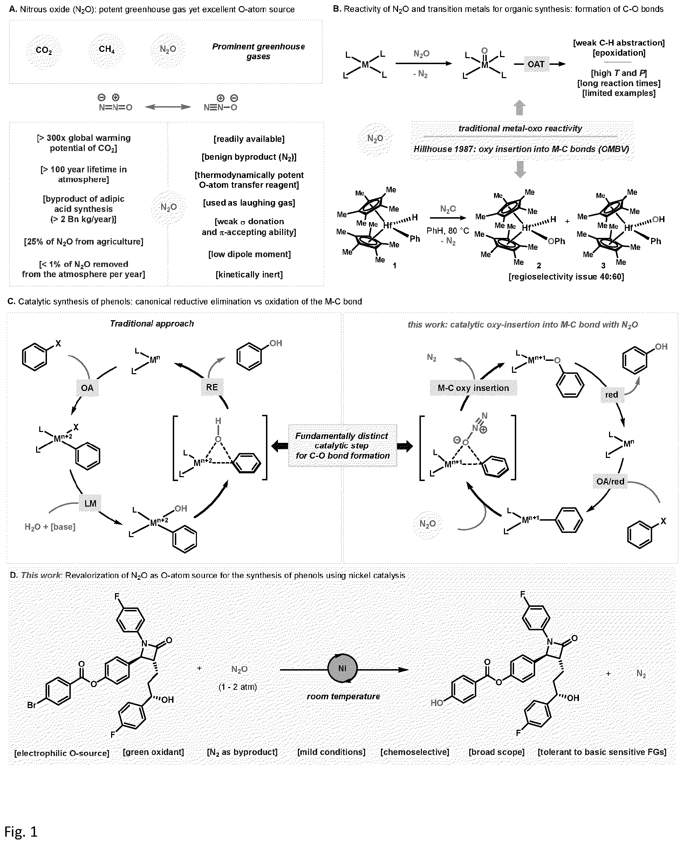

Fig. 1. Relevance of nitrous oxide as green oxidant in the synthesis of phenol derivatives.
   A. Overview of the properties of nitrous oxide.
   **B.** Pioneering examples using N₂O as a reagent for organometallic chemistry.
   **C.** Traditional C(sp²)―O reductive elimination and introduction of O-insertion as a new catalytic step.
   **D.** Introducing the activation of N₂O into a Ni reductive catalytic cycle for crosselectrophile phenol synthesis. OAT: Oxygen Atom Transfer.

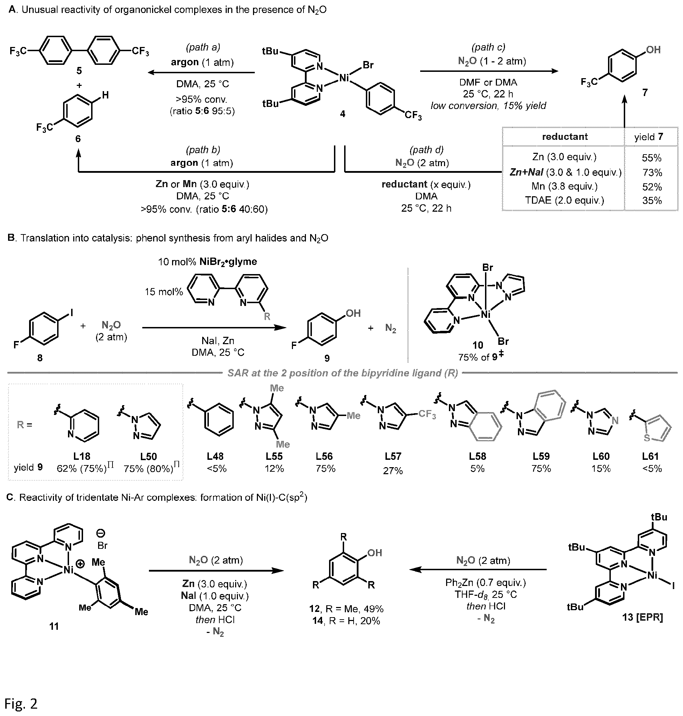

**Fig. 2****.** Design principle and proof-of-concept.
   A. Stoichiometric reactivity using bipyridine supported Ni(II) oxidative addition complexes.
   B. Ligand SAR unlocks the catalytic application of N₂O for the synthesis of phenols.
   C. Stoichiometric reactivity of tridentate terpyridine supported Ni(II) complexes and the involvement of Ni(I) species..
**Fig. 3****.** Revalorization of N₂O as oxidant for the catalytic synthesis of phenols. Scope of aryl iodides. **[N₂]** = N₂ detected by GC-TCD at the end of the reaction. All yields are of isolated pure material. Yield in brackets: ¹H NMR yield calculated using dibromomethane as internal standard. ‡ Denotes use of **terpy L18** as ligand instead of **L50.**
**Fig. 4****.** Inventive reactions
   **A.** Catalytic synthesis of phenols from aryl bromides using N₂O.
   **B.** Exploring the insertion of O-atoms into densely functionalized aryl halides.
   **C.** Using environmentally unfriendly greenhouse gases as building blocks for the synthesis of biologically relevant molecules. All yields are of isolated pure material. See Supplementary information for experimental details. ‡ Denotes use of **terpy L18** as ligand instead of **L50.**

### Experimental Part

To this end, the inventors synthesized the product of oxidative addition **4,** and studied its reactivity with N₂O (Fig. 2A). It is well known that complexes such as **4** rapidly decompose towards homocoupling (5) and protodemetalation **(6)** at 25 °C when dissolved in DMA under argon or N₂ (*path* a). This is exacerbated by the presence of reducing agents such as Zn or Mn (*path b).* Yet, when the inert (N₂ or Ar) atmosphere is replaced by N₂O, the bright red color of the solution of **4** remains, thus pointing towards a slower decomposition rate. After acidic workup, a 15% of phenol **7** is observed. However, when the same reaction is performed in the presence of a reducing agent, substantially higher yields of **7** were observed, with a remarkable 73% yield obtained when a combination of Zn and Nal was used (*path d*)*.*

These results set up the stage for the development of a reductive catalytic protocol based on Ni catalysis using aryl halides. From ligand survey, it was evident that tridentate nitrogenated ligands with the general pattern of 2-substituted bipyridine were crucial to obtain catalytic activity, with terpyridine **(L18)** and 2-pyrazole bipyridine **(L50)** affording the highest yields of **9** (Fig. 2B). SAR analysis of the R group revealed three key features of the ligand for catalytic activity: 1) replacing the N atom for C-H or S prevents catalytic activity **(L48** and **L61);** 2) steric encumbrance in the *ortho* position of the pyrazole unit prevents catalysis **(L55** and **L58);** 3) whereas electron-deficient pyrazole **(L57, L60)** dramatically reduce catalytic activity, electron-rich pyrazole results in comparable yields as for **L50 (L56, L59).** To confirm Ni-ligand ligation in the reaction mixture, complex **10** was prepared and structurally characterized. A 75% yield of **9** as obtained using **10** as catalyst, thus confirming that the pre-ligated complex is catalytically competent. It is important to mention that **L50** represents a new ligand platform within Ni catalysis, with virtually inexistent examples. Since Fig. 2A and 2B suggest that formal Ni(I)―C(sp²) species might be involved, we prepared terpyridine-Ni derivatives such as **11** (Fig. 2C), since greater stability of the corresponding (terpy)Ni(I)-Ar has been noted. Similar to **4,** reaction of **11** in the absence of reducing agent led to no phenol formation. Yet, reaction performed under N₂O in the presence of Zn and Nal afforded the desired mesitol **(12)** in 49% yield upon acidic work up. To further confirm the involvement of such formal Ni(I)-C(sp²) and the need for I in the tridentate system, the (tBu-terpy)Ni(I)-I **(13)** was reacted with Ph₂Zn under N₂O.Despite the reported instability of (terpy)Ni(I)-Ph, a significant 20% of phenol **(14)** was obtained. Altogether, it is suggestive that reduction of Ni(II) species to formal Ni(I) and the aid of I salts in the system are of capital importance to forge the desired C(sp²)-O bond.

Mechanistic investigations on M-Ar oxy insertions for late transition metals (Pd, Ni, Fe) reveal that subtle changes on ligand environment also lead to differences between a M-oxo or a BV pathway for Ar-O bond formation. In our case, we suggest that within the continuum between the two extreme possibilities offered in the OMBV, the oxy-insertion of N₂O in a d⁹ complex lies towards the formation of the M-O bond and N₂, prior to Ar migration.

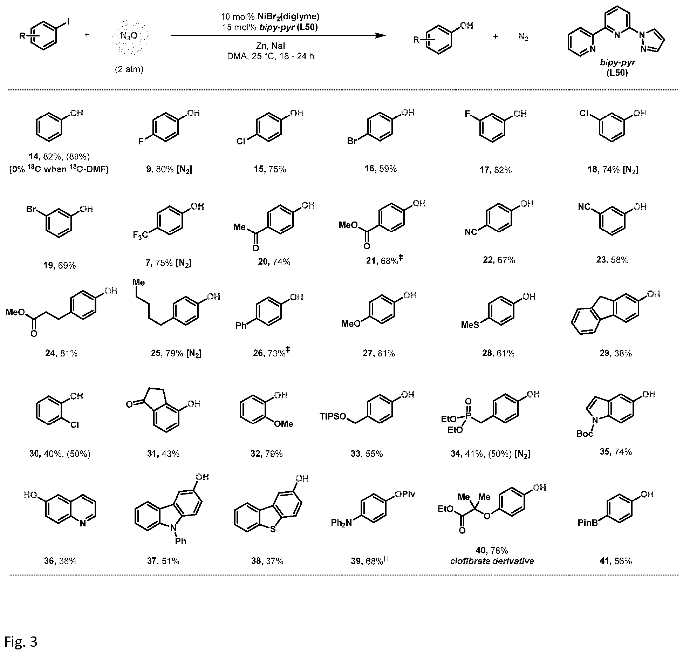

With the optimized catalytic system in hand, a preliminary scope of the aryl halide counterpart was interrogated. As shown in Fig. 3, aryl iodides bearing other halogens in both para- **(9, 15, 16)** and meta-positions **(17-19)** smoothly afforded the corresponding phenol in excellent yields. The presence of electron-withdrawing groups such as CF₃ (7), ketone **(20),** ester **(21** and **24)** or nitrile **(22-23)** posed no difficulty for the C-O bond formation. Electron-donating substituents such as alkyl **(25),** aryl **(26),** or even methoxy and thiomethyl **(27-28)** delivered phenol in good yields. Moreover, fluorene derivative **(29)** featuring benzylic C-H bonds was also amenable for phenol synthesis, albeit in 38% yield. A classical feature of reductive couplings is that steric hindrance at the *ortho*-position can impede reactivity. Indeed, C-O bond formation in indanone **(31)** and 1-chloro-2-iodobenzene **(30)** afforded slightly diminished yields. In contrast to **30, 32** was obtained in 79% yield, illustrating a possible beneficial chelating effect of the ortho OMe and the Ni center. A silylated benzylic alcohol **(33)** or a diethylphosphonate **(34)** were also tolerated in this protocol. Heterocycles such as indole **(35),** quinoline **(36),** carbazole **(37)** or dibenzothiophene **(38)** also afforded the corresponding phenol in good yields. Substrates prone to rapid oxidation after C-O bond formation could be further functionalized *in situ,* as exemplified by the 68% yield obtained for the pivaloyl derivative **39.** Iodide derivative of biologically active clofibrate could be converted to phenol **40** in 78% yield despite the presence of a tertiary α-oxy ester. It is important to mention that this protocol does not require the use of ROH nucleophiles, and hence base-sensitive functionalities such as esters or sensitive amides, which would hydrolyze upon heating, are tolerated. An example of this chemoselectivity is observed in the derivatization of a pinacolborane containing substrate. In this case, phenol **41** was still obtained in 56% yield, thus providing an orthogonal tool to classical oxidation. The observation of 7% yield of sulfoxide in the reaction of **28,** and the low yield obtained for fluorenol **29,** suggest that the oxy-insertion step lies towards the oxo-pathway in the continuum postulated for OMBV-type reactions. It is important to mention that N₂ was detected by GC-TCD in the headspace once the reaction finished (Fig. 1C and 1D). Additionally, when the only alternative source of O was labelled (¹⁸O-DMF), no incorporation of ¹⁸O in **14** was obtained. Altogether, this data supports that the OAT process proceeds via N₂O activation.

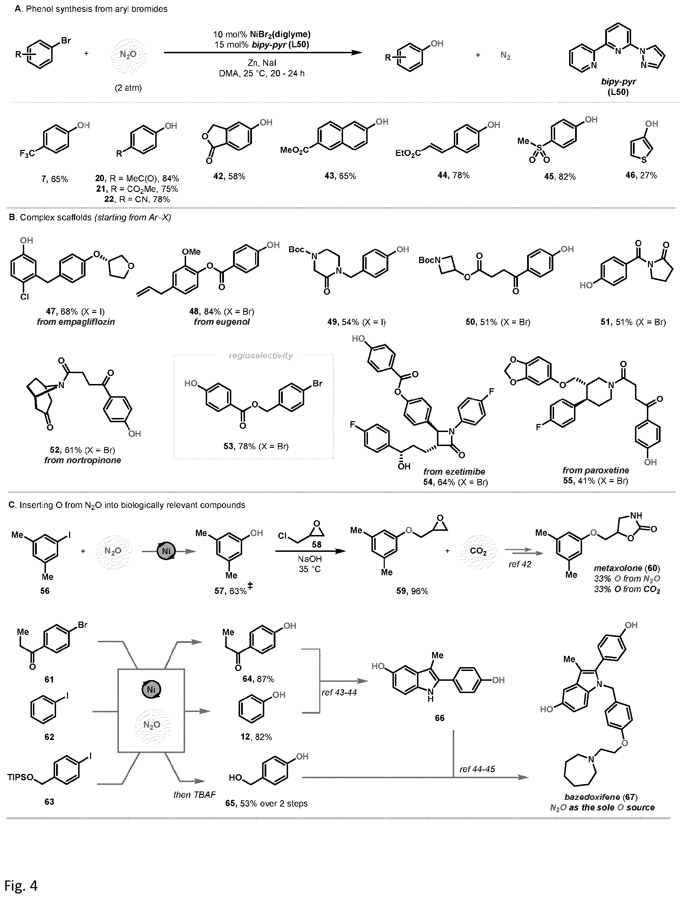

The same optimized reaction conditions for aryl iodides permitted C-O bond formation of more accessible and commercially available aryl bromides. Yet, electron-withdrawing substituents were required to allow C(sp²)―Br cleavage to occur. In this sense, phenols bearing CF₃ **(7),** Ac **(20)** and CN (22) in the para position, as well as paraben **(21),** could be obtained in high yields (Fig 4A). Medicinally relevant phthalide was also smoothly converted to the phenol **(42),** thus providing a 3-step shorter synthesis of this building block compared to the reported process method. Interestingly, phenols derived from π-extended or conjugated systems such as naphthoate **43** or cinnamate **44** were also obtained in 65% and 78% respectively. In contrast to current light-mediated processes, no isomerization of the double bond in **44** was observed. Sulfur-containing scaffolds such as the base-sensitive methyl sulfone **(45)** were obtained in a remarkable 82% yield. Despite the high tendency for self-condensation reported for **46,** a substantial 27% yield could also be isolated.

Complex aryl halides functionalized with sensitive moieties were then tested. For example, empagliflozin derivative, which contains a plethora of weak C-H bonds prone to HAT was smoothly converted to the corresponding phenol **(47)** in excellent yield (Fig 4B). An ester derivative of natural product eugenol afforded the desired phenol **(48)** in a remarkable 84% yield, highlighting the high chemoselectivity of this process over alternative oxidation through metal-oxo pathways. Despite of the tri-phasic nature of the protocol, synthesis of **48** could be scaled up to ca. 1 gram without the need for special setup. Substrates containing saturated N-heterocycles such as piperazinone **49,** azetidine **50,** pyrrolidinone (aniracetam intermediate) **51,** and nortropinone derivative **52** are well tolerated. The requirement of an electron-withdrawing group in order to activate the aryl bromide can be turned into a synthetic advantage, thus permitting regioselective control on the activated aryl bromide **(53,** 78%). Finally, a derivative of high-blood cholesterol drug ezetimibe could be smoothly converted into the corresponding phenol **(54),** without altering the chiral and unprotected secondary alcohol, the ester and strained β-lactam. Similar chemoselectivity can be observed in the conversion of paroxetine derivative **55.** Finally, Fig. 4C illustrates a proof-of-concept of the potential for the revalorization of greenhouse gases for organic synthesis. It is now possible to combine N₂O and CO₂ revalorization strategies and obtain metaxolone **(60),** where 66% of the oxygen atoms originate from waste gaseous feedstock. A more strikingly example is illustrated in the synthesis bazedoxifene **(67),** an anti-breast and -pancreatic cancer drug candidate. Upon successful synthesis of the three phenolic building blocks from the parent halides **(64,** 87%; **12,** 82%; **65,** 53%), and further Fischerindole reaction to access indole **66,** the stage is set to obtain bazedoxifene **(67)** where *all* O *atoms would be originated from N₂O.*

Through a distinct fundamental organometallic step, a catalytic protocol for the revalorization of N₂O as green, mild and chemoselective O-atom insertion reagent for organic synthesis has been unlocked. A mechanistically guided voyage into the reactivity of N₂O with Ni complexes revealed that the unique properties of formally low-valent Ni(I)― aryl permit the O-insertion in an efficient manner. The inert N₂O molecule succumbs to activation under mild conditions toward a mild and selective synthesis of phenols from aryl halides. The catalytic system features an electronically asymmetric tridentate pyrazolyl-bipyridine ligand **(L50)** for the Ni center, which enables a selective C-O bond formation. The reported conditions are simple and robust, allowing C-O bond formation in densely functionalized molecules. Whereas other catalytic protocols capitalize on nucleophilic HO-counterparts, this method represents a unique example of catalytic C-O bond formation with an electrophilic O-atom source, which in turn, can accommodate base-sensitive functionalities. Furthermore, this protocol demonstrates the feasibility of accessing relevant drugs, where N₂O is the sole source of oxygen atoms. Unlocking the catalytic and selective O-insertion into M-C bonds under mild conditions opens the door to the usage of N₂O for the formation of a plethora of O-rich molecules, thus influencing the design of future sustainable catalytic methods.

### Experimental Part

### Instruments

Unless otherwise stated, all manipulations were performed using Schlenk techniques under dry argon in heatgun-dried glassware. Anhydrous solvents were distilled from appropriate drying agents and were transferred under Argon: THF, Et₂O (Mg/anthracene), CH₂Cl₂ (CaH₂), CH₃CN (CaH₂), hexane, toluene (Na/K), benzene-*d*₆ (MS), CD₂Cl₂ (MS), CD₃CN (MS). GC-MS (FID): GC-MS-QP2010 equipped (Shimadzu Europe Analytical Instruments). ESI-MS: ESQ 3000 (Bruker). Accurate mass determinations: Bruker APEX III FT-MS (7 T magnet) or MAT 95 (Finnigan). GC-TCD measurements were performed on Agilent Technologies GC 7890B with a 30 m HP-Plot 5 Å Molsieves column. Melting points were measured with an EZ-Melt Automated Melting Point Apparatus from Stanford Research Systems. NMR spectra were recorded using a Bruker AVIIIHD 300 MHz, Bruker AVIIIHD 400 MHZ, Bruker AVIII 500 MHz or Bruker AVneo 600 MHz NMR spectrometer. The chemical shifts (δ) are given in ppm and were measured relative to solvent residual peak as an internal standard. For ¹H NMR: CDCl₃, δ 7.26; CD₃CN, δ 1.94; CD₂Cl₂, δ 5.32; C₆D₆, δ 7.16. For ¹³C NMR: CDCl₃, δ 77.16; CD₃CN, δ 1.32; CD₂Cl₂, δ 53.84; C₆D₆, δ 128.06. The data is being reported as (s = singlet, d = doublet, t = triplet, q = quartet, quint = quintet, m = multiplet or unresolved bs = broad signal, coupling constant(s) in Hz, integration, interpretation). X-Band EPR spectra were recorded on a Bruker Elexsys E-500 CW X-band spectrometer. The sample tubes of 5 mm outer diameter were placed in a standard TE102 resonator. Low temperature measurements (10-30K) were obtained using an Oxford ESR 900 helium flow cryostat and a Mercury series temperature controller.

### Chemicals

DMF-*d*₇, THF-*d*₈ and toluene-*d*₈ were purchased from Eurisotop, degassed by repeated freeze-pump-thaw cycles, distilled from the proper drying agents, and stored over 4 Å molecular sieves. 4 Å molecular sieves were activated at 200 °C under high vacuum (1 x 10⁻⁴ bar) for 3 days. Unless otherwise noted, all reagents were obtained from commercial suppliers and used without further purification. N₂O was provided by Air Liquide (Distickstoffmonoxid UHP 5.0) containing less than 5 ppm N₂, 1 ppm H₂O and 1 ppm air and O₂

### Examples

### Scope of the catalytic oxidation of aryl halides using N₂O

### General procedure GP1: (using bipy-pyr-L50)

For the scope, oven- and heatgun-dried pressure Schlenks with Teflon screw-cap and equipped with Teflon-coated stir bar were used. These Schlenks were brought to argon filled glovebox, along with a heatgun dried vial. Dried DMA was introduced to the vial. NiBr₂.diglyme (7.1 mg, 0.020 mmol, 10 mol%), bipy-pyr L50 (6.7 mg, 0.030 mmol, 15 mol%), Nal (45 mg, 0.30 mmol, 1.5 equiv), aryl halide (if solid, 0.20 mmol, 1.0 equiv), and zinc (20 mg, 0.30 mmol, 1.5 equiv) were introduced in the pressure Schlenk. Then, outside the glovebox, using a T-connection, the Schlenk was evacuated and refilled with N₂O.This procedure was repeated three times. Then, under 1.5 atm of N₂O, the Schlenk was opened and DMA (0.5 mL) was added using a syringe *while stirring. (Stirring is important to avoid formation of agglomerates.)* If the aryl halide is liquid, it is added at that time using the adapted size of Hamilton syringe. The Schlenk is then closed, and the pressure of N₂O is increased to 1.9 - 2.0 atm. The reaction mixture was kept stirring at 1000 rpm, for 20 to 24 h, at RT.

After the reaction, the Schlenk is carefully opened, and the reaction was first diluted using 1-2 mL of Et₂O or EtOAc depending the solubility of the expected product. The mixture is quenched first with water (the quenching can be exothermic) followed by HCI (1 M), and extracted 3 times with Et₂O (12 to 15 mL). The combined organic layers were washed with brine, dried over MgSO₄, filtered and concentrated under reduced pressure. The crude is purified by column chromatography over silica gel using gradient of solvents.

### General procedure 2: (terpy conditions)

The sole modification for these conditions are highlighted here: NiBr₂.diglyme (7.1 mg, 0.020 mmol, 10 mol%), **terpy L18 (7.0 mg, 0.030 mmol, 15 mol%), Nal (60 mg, 0.40 mmol, 2.0 equiv),** aryl halide (if solid, 0.20 mmol, 1.0 equiv), and **zinc (32.7 mg, 0.500 mmol, 2.5 equiv)** were introduced in the pressure Schlenk.

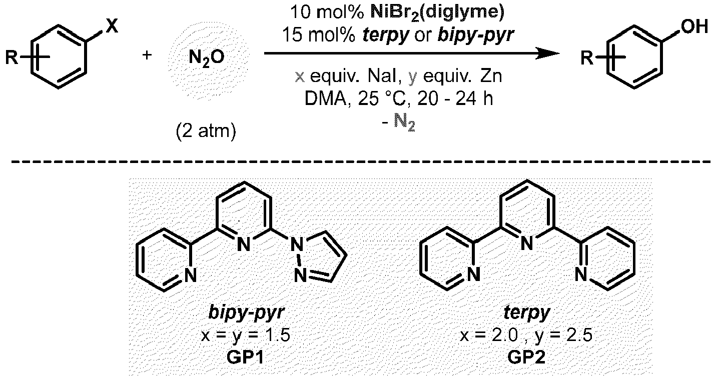

### Scope of aryl iodides

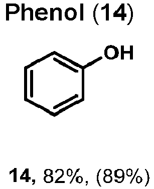

Following general procedure GP1, using phenyl iodide (22 µL, 0.20 mmol, 1.0 equiv) as starting material, 89% NMR yield of the expected product was observed using CH₂Br₂ as internal standard. Purification was performed using Pentane:Et₂O (10:1 to 8:1) as eluent, affording 15.5 mg (0.165 mmol, 82% yield) of phenol **(12)** as a white solid. As a comparison, using **GP2,** 80% NMR yield was observed.
**Rf** (Pentane:Et₂O = 8:2) 0.3

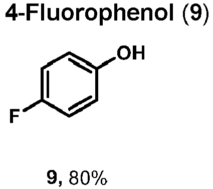

Following general procedure **GP1,** using 4-fluoro-phenyl iodide (23 µL, 0.20 mmol, 1.0 equiv) as starting material, 84% NMR yield was observed, using CH₂Br₂ as internal standard. Purification was performed using Pentane:Et₂O (20:1 to 8:1) as eluent, affording 18.0 mg (0.161 mmol, 80% yield) of 4-fluorophenol (9) was obtained as a pale yellow solid. As a comparison, using **GP2,** 75% NMR yield was observed.

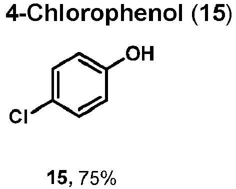

Following general procedure **GP1,** using 1-chloro-4-(iodo)benzene (47.7 mg, 0.200 mmol, 1.0 equiv) as starting material, 19.3 mg (0.150 mmol, 75% yield) of 4-chlorophenol **(15)** was obtained as a light yellow solid after purification using Pentane:Et₂O (19:1 to 10:1) as eluent.

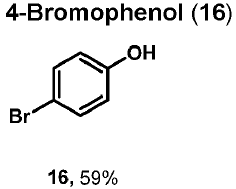

Following general procedure **GP1,** using 1-bromo-4-(iodo)-benzene (56.6 mg, 0.200 mmol, 1.0 equiv) as starting material, 20.4 mg (0.118 mmol, 59% yield) of 4-bromophenol **(16)** was obtained as a light yellow solid after purification using Pentane:Et₂O (19:1 to 10:1) as eluent. 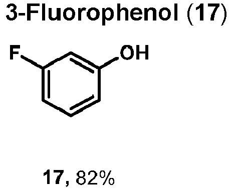 Following general procedure GP1, using 1-fluoro-3-iodobenzene (23.5 µL, 0.200 mmol, 1.0 equiv) as starting material, 18.4 mg (0.164 mmol, 82% yield) of 3-fluorophenol (17) was obtained as a light yellow oil after purification using Pentane:Et₂O (10:1 to 4:1) as eluent.

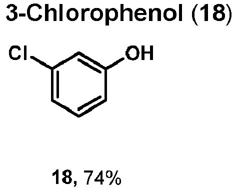

Following general procedure GP1, using 1-iodo-3-(chloro)benzene (24.7 µL, 0.200 mmol, 1.0 equiv) as starting material, 19.0 mg (0.148 mmol, 74% yield) of 3-chlorophenol (18) was obtained as a light yellowish oil after purification using Pentane:Et₂O (10:1 to 4:1) as eluent.

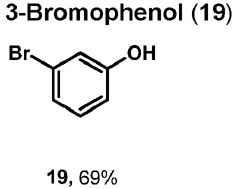

Following general procedure **GP1,** using 1-bromo-3-iodobenzene (25.5 µL, 0.200 mmol, 1.0 equiv) as starting material, 24 mg (0.14 mmol, 69% yield) of 3-bromophenol **(19)** was obtained as a light yellowish oil after purification using Pentane:Et₂O (15:1 to 10:1) as eluent.

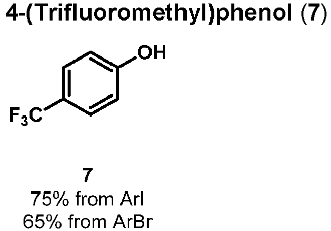

Following general procedure **GP1,** using 1-iodo-4-(trifluoromethyl)benzene (29.4 µL, 0.200 mmol, 1.0 equiv) as starting material, 24.3 mg (0.150 mmol, 75% yield) of 4-(trifluoromethyl)phenol (7) was obtained as a light yellow solid after purification using Pentane:Et₂O (19:1 to 10:1) as eluent.

Following general procedure **GP1,** using 1-bromo-4-(trifluoromethyl)benzene (26 µL, 0.20 mmol, 1.0 equiv) as starting material, 21.1 mg (0.130 mmol, 65% yield) of 4-(trifluoromethyl)phenol (7) was obtained as a light yellow solid after purification using Pentane:Et₂O (19:1 to 10:1) as eluent.

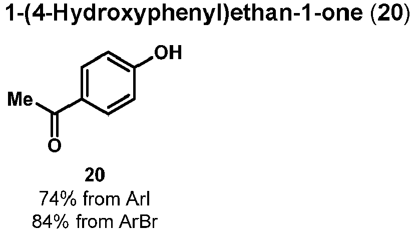

Following general procedure **GP1,** using 4-iodo-acetophenone (49.2 mg, 0.200 mmol, 1.0 equiv) as starting material, 20.1 mg (0.148 mmol, 74% yield) of 1-(4-Hydroxyphenyl)ethan-1-one (20) was obtained as a white solid after purification (2 columns, first one short using Pentane:Et₂O (10:1 to 5:1) then 2^{nd} column using DCM to DCM/EtOAc 95:5 as eluent). Following general procedure **GP1,** using 4-bromo-acetophenone (39.9 mg, 0.200 mmol, 1.0 equiv) as starting material, 22.8 mg (0.167 mmol, 84% yield) of 1-(4-Hydroxyphenyl)ethan-1-one (20) was obtained as a white solid after purification using Pentane:Et₂O (4:1 to 2:1).

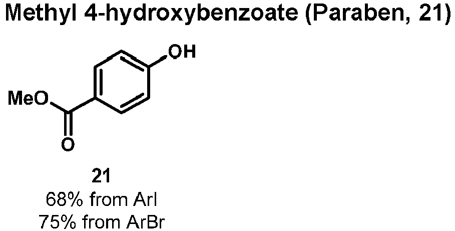

Following general procedure **GP2,** using methyl 4-iodobenzoate (52.4 mg, 0.200 mmol, 1.0 equiv) as starting material, 20.7 mg (0.136 mmol, 68% yield) of methyl 4-hydroxybenzoate (Paraben, **21)** was obtained as a white solid after purification using Pentane:Et₂O gradient (from 9:1 to 7:3) as eluent.

Following general procedure **GP1,** using methyl 4-bromobenzoate (43 mg, 0.20 mmol, 1.0 equiv) as starting material, 22.8 mg (0.150 mmol, 75% yield) of methyl 4-hydroxybenzoate (Paraben, **21)** was obtained as a white solid after purification using Pentane:Et₂O gradient (from 9:1 to 7:3) as eluent.
**Rf** (Pentane:Et₂O = 7:3) 0.2

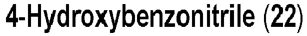

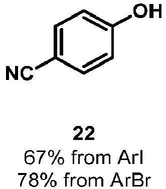

Following general procedure **GP1,** using 4-iodo-benzonitrile (45.8 mg, 0.200 mmol, 1.0 equiv) as starting material, 15.9 mg (0.133 mmol, 67% yield) of 4-hydroxybenzonitrile (22) was obtained as a white solid after purification using DCM:EtOAc (99:1 to 97:3) as eluent. (no HCI was used during workup, and only water)

Following general procedure **GP1,** using 4-bromo-benzonitrile (36.4 mg, 0.200 mmol, 1.0 equiv) as starting material, 18.6 mg (0.156 mmol, 78% yield) of 4-hydroxybenzonitrile (22) was obtained as a white solid after purification using DCM:EtOAc (99:1 to 97:3) as eluent. (no HCI was used during workup, and only water)

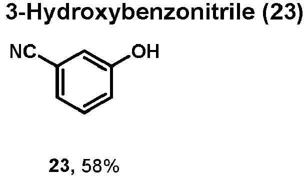

Following general procedure **GP1,** using 3-iodo-benzonitrile (45.8 mg, 0.200 mmol, 1.0 equiv) as starting material, 13.8 mg (0.116 mmol, 58% yield) of 3-hydroxybenzonitrile (23) was obtained as a light yellowish oil after purification using Pentane:Et₂O (9:1 to 3:1) as eluent. *No HCl was used during the workup.*

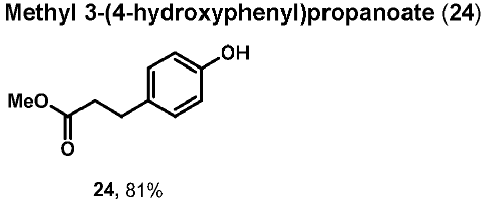

Following general procedure **GP1,** using methyl 3-(4-iodophenyl)propanoate (58 mg, 0.20 mmol, 1.0 equiv) as starting material, 29.1 mg (0.161 mmol, 81% yield) of methyl 3-(4-hydroxyphenyl)propanoate **(24)** was obtained as a light yellow solid after purification using Pentane:Et₂O (7:1 to 3:1) as eluent.
**Rf** (Pentane:Et₂O (6:4)) 0.40

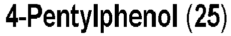

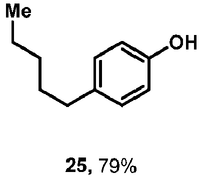

Following general procedure GP1, using 1-iodo-4-(pentyl)benzene (39.7 µL, 0.200 mmol, 1.0 equiv) as starting material, 26.0 mg (0.158 mmol, 79% yield) of 4-pentylphenol (25) was obtained as a light yellow oil after purification using Pentane:Et₂O (95:5 to 9:1) as eluent.
**Rf** (Pentane:Et₂O (9:1)) 0.25

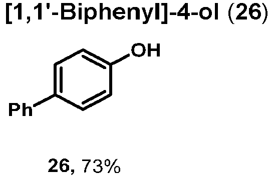

Following general procedure GP2, using 1-iodo-4-(phenyl)benzene (56 mg, 0.20 mmol, 1.0 equiv) as starting material, 24.8 mg (0.146 mmol, 73% yield) of [1,1'-Biphenyl]-4-ol (26) was

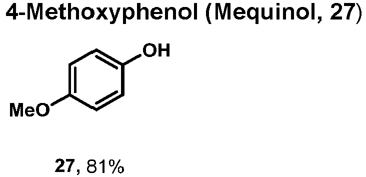

Following general procedure GP1, using 4-iodo-anisole (46.8 mg, 0.200 mmol, 1.0 equiv) as starting material, 20.2 mg (0.163 mmol, 81% yield) of 4-methoxyphenol **(27)** was obtained as a white solid after purification using Pentane:Et₂O (10:1 to 4:1) as eluent.

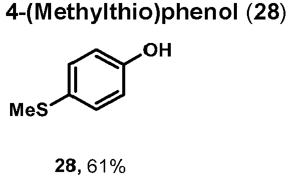

Following general procedure **GP1,** using 4-iodo-thioanisole (50 mg, 0.20 mmol, 1.0 equiv) as starting material, 17.1 mg (0.122 mmol, 61% yield) of 4-(methylthio)phenol **(28)** was obtained as a white solid after purification using Pentane:Et₂O (9:1 to 4:1) as eluent. Detection of 6-7% of the corresponding sulfoxide in the crude NMR.

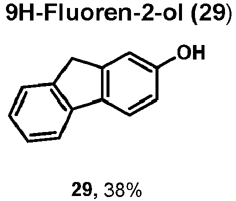

Following general procedure **GP2,** using 2-iodo-9H-fluorene (58.4 mg, 0.200 mmol, 1.0 equiv) as starting material, 14 mg (0.077 mmol, 38% yield) of 9H-Fluoren-2-ol **(29)** was obtained as a white solid after purification using Pentane:Et₂O (10:1 to 8:1) as eluent, followed by a short column using only DCM as eluent to remove colorful impurities.

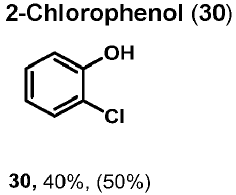

Following general procedure **GP1,** using 1-chloro-2-iodobenzene (24.4 µL, 0.200 mmol, 1.0 equiv) as starting material, 10 mg (0.080 mmol, 40% yield) of 2-chlorophenol **(30)** was obtained as a white solid after purification using Pentane:Et₂O (1:0 to 8:1) as eluent. This compound is relatively volatile, and the isolated yield (40%) is quite lower than the NMR yield on the crude (50%).

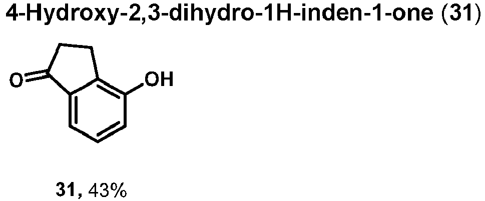

Following general procedure **GP1,** using 4-iodo-2,3-dihydro-1H-inden-1-one (51.6 mg, 0.200 mmol, 1.0 equiv) as starting material, 12.8 mg (0.0864 mmol, 43% yield) of 4-hydroxy-2,3-dihydro-1H-inden-1-one **(31)** was obtained as a white solid after purification using DCM:EtOAc (99:1) as eluent.

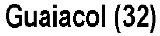

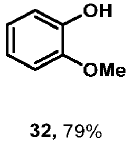

Following general procedure **GP1,** using 2-iodoanisole (26.1 µL, 0.200 mmol, 1.0 equiv) as starting material, 19.6 mg (0.158 mmol, 79% yield) of guaiacol (32) was obtained as a white solid after purification using Pentane:Et₂O (10:1 to 8:1) as eluent.
**Rf** (Pentane:Et₂O (7:3)) 0.45

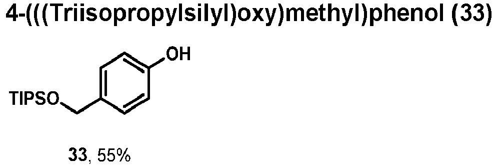

Following general procedure **GP1,** using ((4-iodobenzyl)oxy)triisopropylsilane (78.1 mg, 0.200 mmol, 1.0 equiv) as starting material, 31 mg (0.11 mmol, 55% yield) of 4-(((triisopropylsilyl)oxy)methyl)phenol (33) was obtained as a colorless sticky oil after purification using Pentane:Et₂O (10:1 to 8:1) as eluent.
Rf (Pentane:Et₂O (7:3)) 0.40

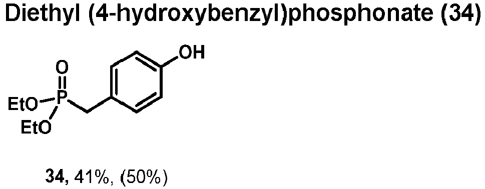

Following general procedure GP1, using diethyl (4-iodobenzyl)phosphonate (70.8 mg, 0.200 mmol, 1.0 equiv) as starting material, NMR yield indicated 50% from the crude, and 20 mg (0.082 mmol, 41% yield) of diethyl (4-hydroxybenzyl)phosphonate (34) was obtained as a solid after tedious purification using DCM:MeOH (1:0 to 95:5) as eluent.

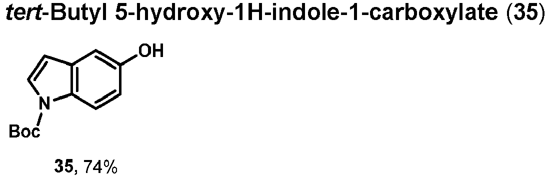

Following general procedure GP1, using *tert*-butyl 5-iodo-1H-indole-1-carboxylate (68.6 mg, 0.200 mmol, 1.0 equiv) as starting material, 34.7 mg (0.149 mmol, 74% yield) of *tert-*butyl 5-hydroxy-1H-indole-1-carboxylate (35) was obtained as a sticky light yellow foam after purification using Hexane:EtOAc (10:1 to 8:1) as eluent.
**Rf** (Hexane:EtOAc (8:2)) 0.4

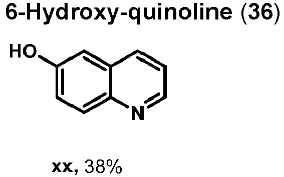

Following general procedure **GP1,** using 6-iodo-quinoline (51 mg, 0.20 mmol, 1.0 equiv) as starting material, 11 mg (0.076 mmol, 38% yield) of 6-hydroxy-quinoline **(36)** was obtained as a white solid after purification using Hexane:EtOAc (2:1 to 1:1) as eluent.
**Rf** (Hexane:EtOAc (1:1)) 0.20

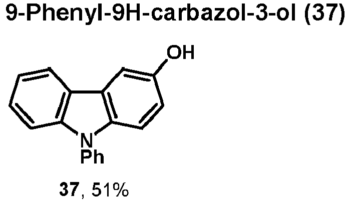

Following general procedure GP1, using 3-bromo-9-phenyl-9H-carbazole (62.2 mg, 0.20 mmol, 1.0 equiv) as starting material, 26.4 mg (0.102 mmol, 51% yield) of 9-phenyl-9H-carbazol-3-ol **(37)** was obtained as a colorless oil after purification using hexanes:EtOAc (5:1) as eluent.
**Rf** (Hexanes:EtOAc (5:1)) 0.20.

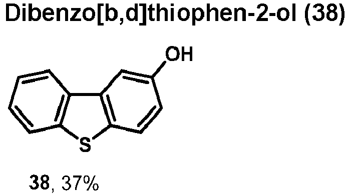

Following general procedure **GP1,** using 2-bromodibenzo[b,d]thiophene (52.4 mg, 0.20 mmol, 1.0 equiv) as starting material, 14.8 mg (0.074 mmol, 37% yield) of dibenzo[b,d]thiophen-2-ol **(38)** was obtained as a white solid after purification using hexanes:EtOAc (5:1) as eluent.
Rf (Hexanes:EtOAc (5:1)) 0.20.

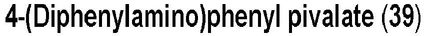

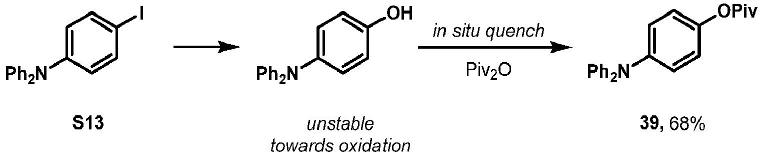

Following general procedure **GP1,** using 4-iodo-triphenylamine **(S13)** (74.2 mg, 0.200 mmol, 1.0 equiv) as starting material, the pressure Schlenk was then carefully open under argon at the end of the first step, and pivalic anhydride (122 µL, 0.600 mmol, 3.0 equiv) was added to the crude reaction mixture and stirred for additional 2 h30 min at 50 °C. After that time, 10 mL of water and drops of NaHCO₃ were added and the crude was extracted 3 times using 12 mL of Et₂O. Then, the combined organic layers were washed using saturated NaHCO₃, dried over MgSO₄, filtered and concentrated under reduced pressure. Column chromatography over silica gel using full Pentane to Pentane:Et₂O (20:1) as eluent deliver 47.0 mg (0.136 mmol, 68% yield) of 4-(diphenylamino)phenyl pivalate (39) as obtained as a colorless oil.

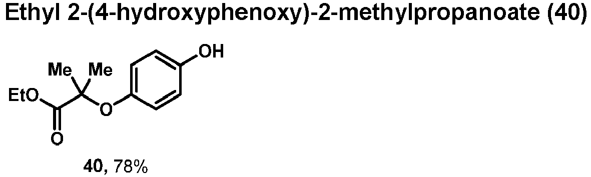

Following general procedure **GP1,** using ethyl 2-(4-iodophenoxy)-2-methylpropanoate (66.8 mg, 0.200 mmol, 1.0 equiv) as starting material, 35.0 mg (0.156 mmol, 78% yield) of ethyl 2-(4-hydroxyphenoxy)-2-methylpropanoate **(40)** was obtained as a off-white solid after purification using Pentane:Et₂O (8:1 to 2:1) as eluent.
Rf (Pentane:Et₂O (7:3)) 0.20

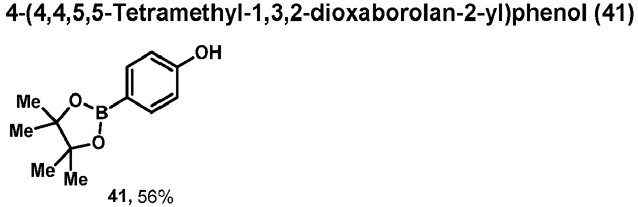

Following general procedure **GP1,** using 4-(4,4,5,5-tetramethyl-1,3,2-dioxaborolan-2-yl)phenyliodide (66 mg, 0.20 mmol, 1.0 equiv) as starting material, 24.7 mg (0.112 mmol, 56% yield) of 4-(4,4,5,5-tetramethyl-1,3,2-dioxaborolan-2-yl)phenol **(41)** was obtained as a white solid after purification using Pentane:Et₂O (10:1 to 8:1) as eluent.
**Rf** (Hexane:EtOAc (3:1)) 0.3

### Scope of aryl bromides

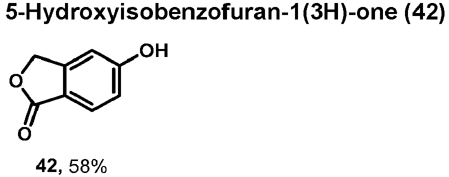

Following general procedure **GP1,** using 5-bromphthalide (42.6 mg, 0.200 mmol, 1.0 equiv) as starting material, 17.5 mg (0.117 mmol, 58% yield) of 5-hydroxyisobenzofuran-1(3H)-one (42) was obtained as a white solid after purification using Hexane:EtOAc (9:1 to 5:1) as eluent.

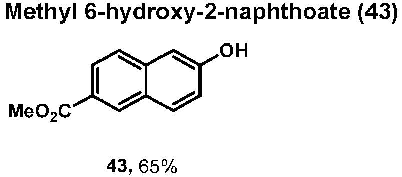

Following general procedure **GP1,** using methyl-6-bromo-2-naphthoate (53 mg, 0.20 mmol, 1.0 equiv) as starting material, 26.2 mg (0.130 mmol, 65% yield) of methyl-6-hydroxy-2-naphthoate **(43)** was obtained as a light brownish - off white solid after purification using DCM:EtOAc (99:1 to 97:3) as eluent followed by trituration in minimal amount of Pentane/DCM.

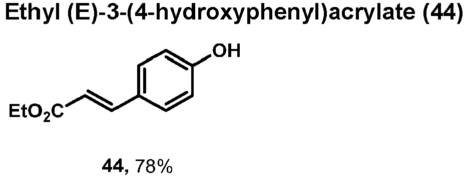

Following general procedure **GP1,** using ethyl (E)-3-(4-bromophenyl)acrylate (37.5 µL mg) as starting material, 30.0 mg (0.156 mmol, 78% yield) of ethyl (E)-3-(4-hydroxyphenyl)acrylate **(44)** was obtained as a white solid after purification using Pentane:Et₂O (6:1 to 3:1) as eluent. No Z isomer was detected.
**Rf** (Pentane:Et₂O (7:3)) 0.2

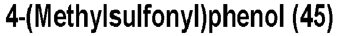

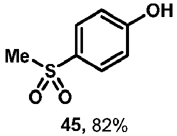

Following general procedure **GP1,** using 1-bromo-4-(methylsulfonyl)benzene (47 mg, 0.20 mmol, 1.0 equiv) as starting material, 28.2 mg (0.164 mmol, 82% yield) of 4-(methylsulfonyl)phenol **(45)** was obtained as a white solid after purification using Hexane:EtOAc (10:1 to 4:1) as eluent.
**Rf** (Hexane:EtOAc (6:4)) 0.5

### Summary:

Catalytic activation and chemical repurposing of potent greenhouse nitrous oxide (N₂O) has long remained a challenge due to its remarkable inertness. Herein, the inventors describe a protocol based on Ni catalysis that unlocks the intrinsic properties of N₂O as a useful O source. The inventors report on the ability of N₂O to undergo O-insertion into Ni-C bonds under mild conditions (room temperature, 1.5 ― 2 bars N₂O), in a manner akin to a Baeyer-Villiger reaction, thus delivering the C-O bond and benign N₂. This fundamental organometallic C-O bond-forming step differs from the canonical reductive elimination and enables a distinct catalytic approach for the conversion of aryl halides into phenols. The process was rendered catalytic by means of a unique bipyridine-pyrazole tridentate ligand for the Ni center, which permits selective C-O insertion in high yields. The method is robust, mild, and highly selective, thus accommodating base-sensitive functionalities as well as permitting phenol synthesis on densely functionalized aryl halides.

## Claims

1. Process for preparing a phenolic hydrocarbon wherein an aryl halogenide is reacted with N₂O in the presence of alkali iodide and Zn in the presence of a Ni-complex having a ligand selected from bipy-pyr and terpy, in a temperature range of 0 °C to 50 °C.

2. Process according to claim 1 wherein the aryl halogenide is selected from:
- monocyclic or polycyclic aromatic hydrocarbons having 6 to 22 carbon atoms,
- monocyclic or polycyclic heteroaromatic hydrocarbons having 5 to 21 carbon atoms, or
- monocyclic or polycyclic aralkyl hydrocarbons having 6 to 34 carbon atoms,
each of which hydrocarbons may be substituted by one or more substituents selected from -OR, -F, -CI, -Br, -I, -NR₂, -CF₃, -CN, -(C=O)-R, -(C=O)-O-R', -(C=O)-NR₂, - (P=O)(OR'))_{2,}, -SO₂R', -SR, -SF₅, -B(OR')₂, wherein R represents hydrogen or an aliphatic hydrocarbon residue having 1 to 12 carbon atoms and wherein R' represents an aliphatic hydrocarbon residue having 1 to 12 carbon atoms.

3. Process according to any of claims 1 to 2, wherein the aryl halogenide is selected from an aryl bromide or aryl iodide.

4. Process according to any of claims 1 to 3, wherein the Ni-complex is prepared in situ from a Ni halogenide selected from NiBr₂, NiI₂ or the corresponding glyme and diglyme adducts thereof which are then complexed with a ligand (L) selected from bipy-pyr and terpy.

5. Process according to any of claims 1 to 3, wherein the Ni-complex is a preformed adduct of the LNiBr₂ or LNiCl₂ wherein L is selected from from bipy-pyr and terpy.

6. Process according to any of claims 1 to 5, wherein the reaction is carried out under a N₂O pressure higher than atmospheric pressure, sufficiently in the range of more than 1,0 to 2,5 bar, preferably in the range of 1,5 to 2,0 bar, more preferably in the range of 1,9 to 2,0 bar.

7. Process according to any of claims 1 to 6, wherein the reaction is carried out in a polar aprotic solvent.
